# FASCICULE DE BREVET EUROPEEN

(11) **EP 0 628 608 B1**
(45) Date de publication et mention de la délivrance du brevet: **22.05.1996**
(21) Numéro de dépôt: 94401239.2
(22) Date de dépôt: 06.06.1994
(51) Int. Cl.: C09C 1/00, C09C 3/06, C08K 3/00, C09D 7/12, C04B 33/14, D21H 17/67, C09D 11/00, A61K 7/00, B32B 9/00

(54) **Procédé de traitement de pigments à base de sulfures de terres rares, pigments ainsi obtenus et leurs utilisations**
Verfahren zum Behandeln von Seltenerdsulfidpigmenten, so erhaltende Pigmente und deren Verwendung
Process for treating rare earth sulfide pigments, thus obtained pigments and their use

(30) Priorité: 09.06.1993 FR 9306899
(43) Date de publication de la demande: 14.12.1994
(73) Titulaire: RHONE-POULENC CHIMIE, 92408 Courbevoie Cédex (FR)
(72) Inventeur: Macaudiere, Pierre, F-92600 Asnières (FR); Morros, Jorge, F-68230 Turckheim (FR); Tourre, Jean-Michel, F-78290 Croissy-sur-Seine (FR); Tressaud, Alain, F-33600 Tessac (FR)
(74) Mandataire: Dubruc, Philippe

(56) Documents cités:
- EP-A- 0 203 838
- CA-A- 2 084 478
- US-A- 4 545 967

## Description

La présente invention concerne, entre autres objets, un procédé de traitement par fluoruration de pigments minéraux colorés à base de sesquisulfures de terres rares en vue notamment d'améliorer la coloration de ces derniers.
Elle concerne également, à titre de produits industriels nouveaux et utiles, des pigments inorganiques colorés à base de sesquisulfures de terres rares présentant des caractéristiques chromatiques améliorées, et susceptibles d'être obtenus en particulier par la mise en oeuvre d'un procédé tel que ci-dessus.
Elle concerne enfin certaines des utilisations possibles des nouveaux pigments selon l'invention pour la coloration de divers matériaux.

On sait que les pigments minéraux de coloration sont déja largement utilisés dans de nombreuses industries notamment dans celles des peintures, des matières plastiques et des céramiques. Dans de telles applications, les propriétés que sont, entre autres, la couleur intrinsèque, le pouvoir de coloration et le pouvoir opacifiant, la stabilité thermique, la stabilité chimique, la dispersabilité (aptitude d'un produit à se disperser correctemment dans un milieu donné), et l'absence de toxicité, constituent autant de critères particulièrement importants à prendre en considération dans le choix d'un pigment convenable.

Les sesquisulfures de terres rares, de formule générale M₂S₃ dans laquelle M désigne au moins une terre rare, sont des produits déja connu en soi et ont fait l'objet de nombreuses publications. Par ailleurs, l'utilisation de ces derniers à titre de pigments colorés pour la coloration de différents matériaux, comme par exemple les plastiques, les peintures et autres, a déja été décrite de manière générale dans la demande de brevet EP-A-0203838, au nom de la Demanderesse. En outre, toujours dans ce même cadre, divers pigments à base de sesquisulfures de terres rares à propriétés améliorées (couleur, stabilité thermique et/ou chimique notamment) ont été par la suite proposés dans les demandes de brevets françaises déposées sous les numéros FR 91/14988 et FR 93/04544, toutes deux également au nom de la Demanderesse. Plus précisément encore, la première demande citée, qui vise essentiellement à l'amélioration des caractéristiques chromatiques des produits, concerne des pigments à base de sesquisulfures de terres rares qui présentent la particularité de contenir en outre au moins un élément alcalin et/ou alcalino-terrreux dont une partie au moins est incluse dans le réseau cristallin desdits sesquisulfures (sesquisulfures dopés), et la deuxième demande, qui vise quant à elle plutôt des améliorations au niveau des propriétés de stabilité thermique et/ou chimique, a trait à des pigments minéraux composites de type core-shell constitués d'un support à base de sesquisulfures dopés tels que précités et d'une couche protectrice à base d'un oxyde transparent déposée à la surface dudit support et enrobant ce dernier.

Poursuivant ses travaux dans le domaine, il est apparu souhaitable à la Demanderesse d'essayer d'améliorer encore certaines des caractéristiques, et en particulier les caractéristiques chromatiques, des pigments à base de sesquisulfures de terres rares connus à ce jour.

Ainsi, l'un des buts que s'est fixée la présente invention réside, entre autres, dans la mise à disposition d'un procédé visant à l'amélioration des propriétés chromatiques des pigments à base de sesquisulfures de terres rares.

La présente invention a également pour but de proposer toute une nouvelle famille de pigments inorganiques colorés présentant, entre autres avantages, des caractéristiques chromatiques tout à fait remarquables, éventuellement une stabilité chimique et thermique améliorées, ainsi qu'une absence de toxicité, lesdits pigments convenant en outre pour la coloration de nombreux matériaux.

Elle vise également à proposer des moyens permettant d'accéder de manière simple, économique, reproductible, contrôlée et compatible avec une production à l'échelle industrielle, à cette nouvelle famille de pigments.

Dans l'exposé qui suit de la présente invention, on entend par sesquisulfures de terres rares tous composés de formule générale M₂S₃ dans laquelle M désigne au moins une terre rare. Par ailleurs, la dénomination terre(s) rare(s) doit s'entendre comme désignant et couvrant, isolément ou en mélange, l'ensemble des éléments appartenant à la famille des lanthanides ayant un numéro atomique compris inclusivement entre 57 et 71, ainsi que l'yttrium de numéro atomique 39. On notera que de tels sesquisulfures de terres rares sont notamment décrits dans la demande de brevet EP-A-0 203 838 précitée, et dont l'enseignement est ici totalement inclus à titre de référence.

En outre, on entend désigner dans ce qui suit par sesquisulfures "dopés" des sesquisulfures de terres rares tels que ci-dessus définis mais contenant en outre un ou plusieurs alcalins, un ou plusieurs éléments alcalino-terreux, ou bien encore des mélanges entre alcalin(s) et alcalino-terreux, lesdits éléments se trouvant pour partie au moins inclus dans le réseau cristallin desdits sesquisulfures. Dans la suite, l'expression "élément dopant" a été utilisée par simplicité pour désigner tant un métal alcalin qu'un métal alcalino-terreux, et elle doit s'entendre comme couvrant toute combinaison entre alcalin(s) et/ou alcalino-terreux, comme souligné ci-dessus. On notera que de tels sesquisulfures dopés, ainsi que leur procédé de préparation, font l'objet de la demande de brevet française FR 91/14988 précitée, et dont l'enseignement est ici totalement inclus à titre de référence. Pour les besoins de l'exposé, une description détaillée de ces produits et de leur mode de préparation sera reprise un peu plus loin.

Ainsi, il a maintenant été trouvé, et c'est ce qui constitue l'un des objets de la présente invention, qu'il est possible d'obtenir des pigments minéraux colorés à base de sesquisulfures de terres rares présentant des propriétés, notamment chromatiques, améliorées, au moyen du nouveau procédé de traitement selon l'invention, ledit procédé étant caractérisé par le fait qu'il consiste à soumettre des pigments à base de sesquisulfures de terres rares, de préférence dopés (au sens donné ci-avant), à un traitement de fluoruration.

Par traitement de fluoruration, on entend ici, de manière très générale, tout traitement capable d'introduire et de fixer des atomes de fluor dans les, et de préférence à la surface des, produits à traiter.

Sans vouloir limiter la présente invention à une quelconque théorie, on peut raisonnablement soutenir que le traitement de fluoruration selon l'invention induit une modification plus ou moins importante de l'état de surface initial des pigments traités, modification qui s'avère probablement à l'origine de l'amélioration de certaines de leurs propriétés, notamment de leur coloration. Cette modification peut être mise en en évidence notamment par des analyses en diffraction X, ESCA et AUGER conduites sur les produits traités ou en cours de traitement.

Les pigments résultant du traitement de fluoruration ci-dessus constituent un autre objet de la présente invention.

Ces nouveaux pigments à base de sesquisulfures de terres rares sont ainsi essentiellement caractérisés par le fait qu'ils contiennent des atomes de fluor.

En outre, ces pigments fluorurés peuvent, de préférence, présenter au moins l'une des caractéristiques suivantes:
- les atomes de fluor sont distribués selon un gradient de concentration décroissant de la surface au coeur desdits sesquisulfures.
- les atomes de fluor sont majoritairement répartis à la périphérie externe desdits sesquisulfures. On entend ici par périphérie externe une épaisseur de matière, mesurée à partir de la surface de la particule, de l'ordre de quelques centaines d'Angströms. On entend en outre par majoritairement que plus de 50% des atomes de fluor présents dans le sesquisulfure se trouvent dans ladite périphérie externe.
- le pourcentage en poids des atomes de fluor présents dans le sesquisulfure n'excède pas 10%, et de préférence 5%.
- les atomes de fluor sont présents sous la forme de composés fluorés ou sulfofluorés, en particulier sous la forme de fluorures de terres rares ou de sulfofluorures (thiofluorures) de terres rares.

Selon un mode particulier de réalisation des nouveaux pigments selon la présente invention, les pigments fluorurés issus de l'étape de fluoruration peuvent en outre, par la suite, être encapsulés par des oxydes transparents pour donner des particules composites de type core-shell selon une technique telle que décrite dans la demande de brevet française FR 93/04544 précitée et dont l'enseignement est ici totalement inclus à titre de référence. Pour les besoins de l'exposé, une description détaillée de ce procédé d'encapsulation, et des produits résultants, sera à nouveau donnée par la suite. Les pigments fluorés ainsi encapsulés présentent, par rapport aux produits correspondants non encapsulés, une stabilité chimique et une stabilité thermique améliorées, voire, dans certains cas, une coloration améliorée.

Le procédé selon l'invention consiste donc, dans la pratique, à mettre en contact et à faire réagir un sesquisulfure de terres rares avec un agent de fluoruration.

Cette fluoruration peut être opérée selon toute technique connue en soi.

En particulier, l'agent de fluoruration peut être liquide, solide ou gazeux. De préférence, on opère sous des conditions de traitement où l'agent de fluoruration est liquide ou gazeux.

A titre d'exemples d'agents fluorurants convenant pour la mise en oeuvre du traitement selon l'invention, on peut plus particulièrement citer le fluor F₂, les fluorures d'halogènes, le fluorure d'ammonium, les fluorures de gaz rares, le fluorure d'azote NF₃, le fluorure de bore BF₃, le tétrafluorométhane, l'acide fluorhydrique HF.

Dans le cas d'un traitement sous atmosphère fluorante, l'agent fluorurant peut être utilisé pur ou en dilution dans un gaz neutre, par exemple de l'azote.

De préférence, les pigments à traiter se trouvent initialement à l'état d'une poudre fine et homogène, et ceci de manière à faciliter le traitement de fluoruration. On notera que le procédé selon l'invention présente pour avantage supplémentaire, entre autres, de ne pas ou peu perturber la granulométrie des particules constituant le pigment initial.

Les conditions de réaction sont choisies de préférence de manière telle que ledit traitement n'induise une fluoruration qu'en surface du matériau (conditions douces). A cet égard, la conduite d'une fluoruration jusqu'au coeur du matériau n'apporte pas de résultats substantiellement améliorés par rapport à une fluoruration essentiellement de surface. D'une manière pratique, on peut suivre et contrôler expérimentalement le degré d'avancement de la réaction de fluoruration par exemple en mesurant l'évolution de la prise de masse des matériaux (prise de masse induite par l'introduction progressive du fluor).

Divers modes particuliers, et les conditions de réaction y afférentes, relatifs à la mise en oeuvre de procédés de fluoruration conformes à l'invention seront présentés dans les exemples donnés ci-après. Il demeure bien entendu que la présente invention n'est pas limitée à ces différents exemples de mise en oeuvre, mais qu'elle englobe toutes les variantes possibles. En particulier, des modifications de certains paramètres expérimentaux tels que pression, température, dilution et nature de l'agent fluorurant; durée et régime (dynamique ou statique) de la fluoruration; etc..., permettant une optimisation de la coloration des pigments à base de sesquisulfures de terres rares, peuvent être apportées dans la mise en oeuvre du procédé conforme à l'invention sans se départir toutefois du cadre et de l'esprit de cette invention.

On notera que le procédé de traitement selon l'invention est d'application et de portée générale, c'est à dire qu'il convient au traitement et à la préparation de toute une gamme de différents sesquisulfures de terres rares, dès lors qu'une amélioration significative des propriétés de coloration de ces derniers est recherchée. Bien entendu, le pigment initial à base de sesquisulfures de terres rares détermine et conditionne la coloration finale du pigment fluoruré correspondant conforme à l'invention; en d'autres termes, l'obtention d'un pigment fluoruré devant présenter une couleur finale donnée passe naturellement par l'utilisation d'un pigment initial qui, originellement, présente cette même couleur. Ainsi, le traitement selon l'invention ne modifie substantiellement pas la nature et la composition chimique d'une part et la couleur intrinsèque de base d'autre part, du pigment originel. Il en résulte que les pigments selon l'invention ne se distinguent des pigments connus de départ (avant traitement) essentiellement que par la présence de fluor d'une part, et ceci de la manière définie ci-avant dans la description, et une coloration améliorée d'autre part. Enfin, on notera que le procédé de traitement selon l'invention présente pour autre avantage majeur de ne pas modifier ou perturber les autres propriétés pigmentaires originelles de base des produits de départ, à savoir principalement leur pouvoir de coloration, leur pouvoir opacifiant et leur dispersabilité dans des matières à colorer.

Les pigments susceptibles d'être traités et/ou obtenus dans le cadre de la présente invention vont maintenant être développés un peu plus en détails.

Ainsi, le procédé selon l'invention convient particulièrement bien au traitement et à l'obtention de pigments à base de sesquisulfures de terres rares de type dopés (présence d'un ou plusieurs éléments alcalins et/ou alcalino-terreux dans le réseau cristallin du sesquisulfure), tels que décrits dans la demande de brevet FR 91/14988, et qui possèdent déja intrinsèquement de nombreuses propriétés avantageuses, notamment une coloration nettement améliorée par rapport aux pigments correspondants non dopés. Le procédé selon l'invention convient aussi néanmoins au traitement des sesquisulfures non dopés, tels que ceux notamment décrits dans la demande de brevet EP-A- 0 203 838.

L'élément dopant peut être présent sous différentes formes dans le pigment. Toutefois, selon l'invention, il est de préférence essentiellement présent sous une forme combinée avec les sesquisulfures de terres rares. Dans ce cas, I'élément dopant est alors lié de façon irreversible aux sesquisulfures, en ce sens que, par exemple, même des lavages très poussés de ces derniers ne permettent pas de l'éliminer. De tels lavages peuvent conduire par contre à l'élimination des éventuels sulfures et/ou polysulfures d'alcalin(s) ou d'alcalino-terreux présents à la surface des pigments (avant fluoruration), et donc non liés de façon irreversible à ces derniers.

Sans vouloir se ramener ou se limiter à une quelconque théorie concernant les causes et les effets du dopage, on peut néanmoins ici avancer l'explication probable suivante:
on sait que les sesquisulfures de terres rares M₂S₃ cristallisent selon une structure cristallographique de type Th₃P₄, qui présente des lacunes au niveau du réseau des cations; cette structure lacunaire peut être symbolisée en donnant aux sesquisulfures la formule M_{10,66} [ ]_{1,33} S₁₆ (voir notamment à ce sujet, W.H. ZACHARIASEN, "Crystal Chemical Studies of the 5f-Series of Elements. The Ce₂S₃-Ce₃S₄ Type of Structure", Acta Cryst. (1949), 2, 57).

Or, il a été trouvé par la présente Demanderesse que des éléments alcalins et/ou alcalino-terreux peuvent être introduits dans ces lacunes cationiques, jusqu'à saturation ou non de ces dernières. La présence de l'élément dopant au sein des pigments obtenus ou mis en oeuvre dans le cadre de l'invention peut ainsi être mise en évidence par simple analyse chimique. Par ailleurs, les analyses en diffraction X montrent qu'il y a conservation de la phase cristalline en Th₃P₄ du sesquisulfure, avec dans certains cas, une modification plus ou moins importante des paramètres de maille, fonction à la fois de la nature et de la quantité de l'élément dopant introduit.

De façon tout à fait inattendue et surprenante, il s'avère que cette insertion au sein du réseau cristallin du sesquisulfure confère à ce dernier des caractéristiques chromatiques nettement améliorées par rapport à tous les sesquisulfures (non dopés) de terres rares connus à ce jour. En outre, la présence de cet élément dopant peut avoir pour effet bénéfique de stabiliser aux hautes températures la structure cristalline du sesquisulfure considéré, et donc de conserver la couleur désirée dans une plus grande plage de température.

Selon un mode de réalisation préféré des pigments fluorurés selon l'invention, l'élément dopant présent dans le sesquisulfure est un élément alcalin et est par exemple choisi, seul ou en mélange, parmi le lithium, le sodium et le potassium. De manière encore plus préférentielle, l'alcalin est le sodium.
Parmi les éléments alcalino-terreux utilisables selon l'invention, on peut plus particulièrement citer le magnésium, le calcium, le baryum et le strontium.

Selon une autre caractéristique préférée desdits pigments, la quantité molaire en alcalin(s) et/ou alcalino-terreux présent(s) dans le pigment est au moins égale à 0,1%, et avantageusement comprise entre 5% et 50%, de la quantité molaire en la ou les terres rares présentes dans ce pigment.

Comme indiqué précédemment, la nature de la ou des terres rares, ainsi que le type de réseau cristallin du sesquisulfure, sont choisis en fonction de la couleur que l'on désire obtenir pour le pigment après fluoruration. Par ailleurs, dans tous les cas, on observe que, pour un élément dopant convenablement sélectionné, les pigments fluorurés selon l'invention présentent alors des colorations beaucoup plus intenses que celles des sesquisulfures correspondants non dopés par un élément alcalin et/ou alcalino-terreux; par sesquisulfure correspondant, on entend le sesquisulfure contenant la ou les mêmes terres rares et présentant la même forme cristallographique.
Pour un sesquisulfure de terre rare donné, et donc de coloration donnée, on peut donc ainsi accéder, après de simples essais de routine, à toute une gamme de pigments de couleurs améliorées, et ceci en jouant simplement sur la nature et/ou sur la concentration en élément dopant dans le pigment.

On donne ci-dessous des exemples de couleurs auxquelles il est possible d'aboutir avec les pigments fluorurés selon l'invention, et ceci en fonction de la nature du pigment originel mis en jeu (dopé ou non), cette liste n'étant bien entendu donnée qu'à titre purement illustratif et donc nullement limitatif:
- les pigments traités qui sont à base de sulfures de cérium ont une couleur variant du brun au rouge selon les conditions de préparation, en particulier la température de calcination. Ils sont bruns ou rouges sang selon que l'on ait la phase Ce₂S₃ β orthorombique (J.C.P.D.S. 20 269) ou la phase Ce₂S₃ γ cubique (J.C.P.D.S. 27 104)
- avec le lanthane, on obtient des pigments jaunes, et ceci avec une structure La₂S₃ cubique (J.C.P.D.S. 25 1041)
- une coloration verte peut être obtenue avec le néodyme, et une coloration vert-jaune avec le praséodyme. Les pigments présentent alors respectivement la structure Nd₂S₃ cubique (J.C.P.D.S. 26 1450) et la structure Pr₂S₃ cubique (J.C.P.D.S. 27 481)
- on dispose d'un pigment jaune-marron avec le dysprosium de structure Dy₂S₃ cubique (J.C.P.D.S. 26 594)
- des pigments présentant différentes nuances de marron peuvent aussi être obtenus : ocre avec le terbium de structure Tb₂S₃ cubique, brun avec l'erbium de structure Er₂S₃ monoclinique (J.C.P.D.S. 21 324) et beige foncé avec l'yttrium de structure Y₂S₃ monoclinique (J.C.P.D.S. 22 996)
- d'autres exemples de couleurs que l'on peut obtenir sont enfin : brun-gris avec le samarium de structure Sm₂S₃ cubique (J.C.P.D.S. 26 1480), brun-vert avec le gadolinium de structure Gd₂S₃ γ cubique (J.C.P.D.S. 26 1424), vert-or avec le thullium de structure Tm₂S₃ monoclinique (J.C.P.D.S. 30 1364).

La coloration intrinsèque tant des pigments dopés originels que des pigments dopés fluorurés selon l'invention, peut par ailleurs être quantifiée au moyen des coordonnées chromatiques L*, a* et b* données dans le système CIE 1976 (L*,a*,b*) tel que défini par la Commission Internationale d'Eclairage et répertorié dans le Recueil des Normes Françaises (AFNOR), couleur colorimétrique n° X08-12 (1983). Elles sont déterminées au moyen d'un colorimètre commercialisé par la Société Pacific Scientific. La nature de l'illuminant est D65. La surface d'observation est une pastille circulaire de 12,5 cm de surface. Les conditions d'observation correspondent à une vision sous un angle d'ouverture de 10°. Dans les mesures données, la composante spéculaire est exclue.

L* donne une mesure de la réflectance (nuance clair/sombre) et varie ainsi de 100 (blanc) à 0 (noir).
a* et b* sont les valeurs des tendances colorées:
a* positif = rouge
a* négatif = vert
b* positif = jaune
b* négatif = bleu

L* représente donc la variation du noir au blanc, a* la variation du vert au rouge et b* la variation du bleu au jaune.

Ainsi, selon la présente invention, et à titre seulement d'exemple, quand la terre rare est le cérium et que le sesquisulfure est sous sa forme cristallographique γ cubique, on dispose de pigments présentant les coordonnées trichromatiques tout à fait remarquables suivantes:
- L* au moins égal à 30, et notamment comprise entre 30 et 60,
- a* au moins égal à 30, et notamment comprise entre 35 et 65,
- b* compris généralement entre 0 et 55.

De telles coordonnées, et en particulier la coordonnée chromatique a*, correspondent à une couleur rouge intense tout à fait exceptionnelle pour un sulfure de cérium Ce₂S₃ γ cubique, et est équivalente voire supérieure à celle des pigments rouges de référence, à savoir le sélénure de cadmium et le sulfosélénure de cadmium. En outre, l'un des avantages d'un tel pigment est qu'il ne présente pas les problèmes de toxicité liés à la présence de métaux lourds, comme cela est généralement le cas dans les pigments de l'art antérieur.

Comme le mettront clairement en évidence les exemples concrêts donnés ci-après, le traitement de fluoruration selon l'invention convient particulièrement bien pour améliorer de manière substantielle et significative la coordonnée chromatique a* (tendance rouge) des pigments à base de sesquisulfure de cérium Ce₂S₃ γ cubique dopé par du sodium.

Comme indiqué précédemment dans la description, on va maintenant rappeler ci-dessous les détails d'un procédé industriel particulièrement avantageux convenant à la synthèse des sesquisulfures dopés par des alcalins et/ou des alcalino-terreux tels que décrits ci-avant, ces derniers produits constituant finalement l'une des matières premières préférées essentielles à la préparation des pigments fluorurés selon l'invention. On peut d'ores et déja noter que le procédé qui suit, qui fait par ailleurs l'objet de la demande de brevet FR 91/14988 précitée, convient particulièrement bien, entre autres, pour accéder à des compositions dans lesquelles le sesquisulfure de terres rares se présente sous une forme cristalline cubique, et notamment γ cubique.

Ce procédé consiste à réaliser un mélange initial contenant au moins un composé de terre rare, du soufre et au moins un composé d'un élément alcalin et/ou alcalino-terreux (élément dopant), à chauffer ledit mélange initial jusqu'à l'obtention de la phase sesquisulfure désirée, et ceci sous une atmosphère non oxydante, de préférence réductrice, puis à refroidir le mélange ainsi traité.

Selon une variante avantageuse de ce procédé, le chauffage du mélange initial est réalisé en présence d'un agent réducteur. La quantité d'agent réducteur ajoutée est alors déterminée de manière à maintenir une pression partielle d'oxygène très basse dans le réacteur. Ainsi, la quantité d'agent réducteur mise en oeuvre est avantageusement suffisante pour consommer l'oxygène libre et/ou combiné contenu dans le mélange initial.

Dans un premier mode possible de réalisation de cette variante, un agent réducteur est ajouté directement au mélange initial. Cet agent est généralement à base de carbone, tel que, par exemple, le graphite, le coke, la lignite ou bien encore un composé organique générant du carbone par chauffage. Ce peut être aussi un réducteur métallique, par exemple l'aluminium.

Selon un second mode possible de réalisation de cette variante, l'agent réducteur est contenu dans le gaz formant l'atmosphère non oxydante. On réalise alors avantageusement un balayage du mélange initial avec un gaz non oxydant, de préférence un gaz inerte, contenant un agent réducteur tel que par exemple l'hydrogène ou le monoxyde de carbone CO. On peut ainsi mettre en oeuvre un mélange d'hydrogène avec un gaz inerte, tel qu'un mélange argon/hydrogène ou azote/hydrogène, ou bien encore un mélange argon/CO ou azote/CO. Ce balayage peut également être réalisé par de l'hydrogène ou du monoxyde de carbone seuls.

Avantageusement, lors de la montée en température, on maintient le mélange à une température intermédiaire, notamment comprise entre 250°C et 500°C, avant de porter celui-ci à la température correspondant à la formation du sesquisulfure désiré. Ce maintien à une température intermédiaire est réalisé pendant une durée comprise généralement entre 15 minutes et 1 heure.

Les composés de terres rares convenables pour la mise en oeuvre du procédé ci-dessus sont par exemple choisis dans le groupe constitué par les composés oxycarbonés de terres rares, les sulfates, les oxydes de terres rares. A titre de composés oxycarbonés de terres rares, on peut citer notamment les carbonates, oxalates, acétates, malonates, tartrates de terres rares.

Concernant les composés d'alcalins ou d'alcalino-terreux utilisables dans ce même procédé, on peut citer par exemple les oxydes, les sulfures ou polysulfures, les sulfates, les composés oxycarbonés tels qu'oxalates, carbonates ou acétates d'alcalins ou d'alcalino-terreux. De préférence, on met en oeuvre des carbonates de ces éléments.

La quantité d'élément alcalin ou alcalino-terreux ajoutée est déterminée pour avoir un rapport molaire : élément dopant / terre(s) rare(s) compris généralement entre 0,05 et 0,5, et de préférence entre 0,15 et 0,30 dans le mélange initial.

Par ailleurs, la quantité de soufre présent dans le mélange initial est déterminée pour avoir un rapport molaire : soufre / terre(s) rare(s) supérieur ou égal à 1,5 et de préférence supérieur à 2.
Le soufre peut être introduit sous forme libre (soufre élémentaire solide ou gazeux) ou sous forme d'un composé soufré précurseur, par exemple Na₂S, H₂S ou CS₂.
De préférence, on utilise soit du soufre élémentaire à l'état solide soit CS₂ gazeux.

Le mélange initial peut bien entendu comprendre plusieurs composés de terres rares et/ou d'alcalins et/ou d'alcalino-terreux, comme souligné ci-avant.

Le mélange initial ainsi obtenu est ensuite chauffé à une température et pendant un temps suffisants pour obtenir la phase sesquisulfure désirée, ce temps étant généralement d'autant plus court que la température est élevée. Cette température dépend bien entendu du sesquisulfure considéré.

Avantageusement, le mélange est chauffé à une température supérieure à 900°C, généralement comprise entre 1000°C et 1400°C, de préférence 1150-1300°C, et ceci pendant au moins 30 minutes, de préférence entre 30 minutes et deux heures.

Le produit ainsi obtenu peut ensuite éventuellement être soumis à un ou des lavages, par exemple à l'eau, pour diminuer la teneur en alcalin(s) et/ou alcalino-terreux non liés.

Si nécessaire, le produit obtenu peut enfin être broyé (broyage à jet d'air ou autre) pour obtenir un diamètre moyen de grains compris entre 0,2 µm et 5 µm. Toutefois, selon le procédé tel qu'exposé ci-dessus, on arrive généralement à cette granulométrie sans avoir à broyer le produit, ce qui constitue un avantage très important d'un point de vue économique.

Le produit obtenu présente alors une très bonne pureté phasique (absence d'oxysulfure notamment) et des cooordonnées chromatiques remarquablement élevées dans la couleur spécifique du sesquisulfure de terre rare considéré.

Comme souligné précédemment, ces produits (sesquisulfures de terres rares dopés) sont alors destinés à constituer l'une des matières premières susceptibles d'être traitées conformément au procédé selon la présente invention en vue d'aboutir à des pigments fluorurés à propriétés, notamment chromatiques, améliorées et objets de la présente invention.

On revient donc maintenant plus spécifiquement à l'exposé des pigments selon la présente invention.

Comme indiqué ci-avant au début de la présente description, un mode particulier de réalisation et de présentation des nouveaux pigments fluorurés selon l'invention consiste, par la suite, à encapsuler, selon une technique telle que déja décrite dans la demande de brevet FR 93/04544 précitée, lesdits pigments par une couche d'oxydes transparents, et ceci dans le but notamment d'obtenir des pigments fluorurés présentant, par rapport aux produits fluorurés correspondants non encapsulés, une stabilité chimique et une stabilité thermique améliorés, voire, dans certains cas, une coloration encore améliorée.

Selon cette technique d'encapsulation, dont les détails seront, pour les besoins de l'exposé, rappelés ci-après, on obtient ainsi un nouveau pigment composite coloré de type core-shell constitué plus précisément:
- d'un support (ou coeur, ou noyau) à base d'au moins un sesquisulfure de terres rares fluoruré (dopé ou non) tel qu'obtenu et défini précédemmment,
- et d'une couche (ou écorce, ou enveloppe) à base d'au moins un oxyde transparent déposée à la surface dudit support et enrobant ce dernier.

Bien entendu, certaines variantes autour de cette structure sont possibles. En particulier, la couche périphérique enrobant la particule peut ne pas être parfaitement continue ou homogène. Toutefois, de préférence, les pigments selon l'invention sont constitués d'une couche de revêtement uniforme et d'épaisseur contrôlée d'oxyde transparent, et ceci de manière à ne pas altérer la couleur originelle du support avant enrobage.

Par oxyde transparent, on entend ici un oxyde qui, une fois déposé sur un support sous la forme d'une pellicule plus ou moins fine, n'absorbe que peu ou pas du tout les rayons lumineux dans le domaine du visible, et ceci de manière à ne pas ou peu masquer la couleur intrinsèque d'origine dudit support. En outre, il convient de noter que le terme oxyde, qui est utilisé ici par simple commodité, doit être entendu comme couvrant également des oxydes de type hydratés.
Ces oxydes, ou oxydes hydratés, peuvent être amorphes et/ou cristallisés.
A titre d'exemple de tels oxydes, on peut plus particulièrement citer l'oxyde de silicium (silice), l'oxyde d'aluminium (alumine), l'oxyde de zirconium (zircone), l'oxyde de titane, le silicate de zirconium ZrSiO₄ (zircon) et les oxydes de terres rares. Parmi ceux-ci, on préfére mettre en oeuvre ceux ne présentant pas de toxicité officiellement reconnue pour l'homme et/ou son environnement.
Selon un mode préféré de réalisation des pigments composites selon la présente invention, la couche enrobante est à base de silice. De manière encore plus avantageuse, cette couche est essentiellement, et de préférence uniquement, constituée de silice.

Les nouveaux pigments composites ci-dessus peuvent être utilisés avec succès en tant que pigments colorés en raison de leurs nombreuses et multiples propriétés avantageuses, telles que notamment un très large éventail d'excellentes couleurs intrinsèques, un très bon pouvoir de coloration, un très bon pouvoir opacifiant, une très bonne dispersabilité notamment dans les plastiques, une très bonne stabilité thermique à des températures pouvant aller jusqu'à 500°C, ainsi qu'une bonne stabilité chimique dans des milieux comme l'eau (à pH neutre, basique ou faiblement acide) et les solvants organiques.

Le procédé de synthèse (encapsulation) des pigments composites selon l'invention va maintenant être développé un peu plus en détails.

Ce procédé comprend les étapes essentielles suivantes:
(i) on met en contact un support minéral tel que ci-dessus défini et un précurseur de l'oxyde transparent désiré,
(ii) on précipite l'oxyde transparent,
(iii) et enfin on sépare le pigment composite obtenu du milieu de réaction.

Globalement, un tel procédé repose sur le principe d'une précipitation chimique d'un précurseur d'oxyde transparent opérée "in situ" dans un milieu constitué d'une dispersion (ou suspension) du support minéral précité, ledit oxyde précipité se déposant alors à la surface de chacune des particules dudit support minéral.
Il en résulte l'obtention directe dans le milieu de réaction de particules composites (ou pigments) qui correspondent au support fluoruré originel mais sous une forme parfaitement encapsulée par une couche protectrice externe, d'épaisseur contrôlée, d'oxyde transparent.

Ce procédé d'encapsulation présente pour avantage, entre autres, de ne pas ou peu perturber d'une part la granulométrie des particules constituant le support initial avant enrobage, ainsi que, d'autre part, les excellentes caractéristiques chromatiques intrinsèques attachées à ces dernières, lesdites caractéristiques pouvant même s'en trouver sensiblement améliorées; en outre, I'écorce ainsi constituée à la surface desdites particules initiales permet d'aboutir à des pigments dont les propriété de stabilité thermique et chimique sont nettement améliorées.

Les techniques de précipitation qui peuvent être utilisées dans le cadre du procédé d'encapsulation sont multiples et varient généralement avec la nature de l'oxyde transparent recherché. Ce qui suit n'est donc donné qu'à titre pûrement illustratif et nullement limitatif. On notera seulement qu'il existe une exigence générale commune à respecter pour la bonne marche de tous ces procédés de précipitation, à savoir qu'il convient dans toute la mesure du possible d'éviter que les sesquisulfures de terres rares fluorurés utilisés à titre de support ne soient mis en contact, avant ou pendant l'étape de précipitation, avec un milieu trop acide qui pourrait provoquer la décomposition de ces derniers.

L'encapsulation va tout d'abord être décrite dans le cas particulier et préféré où l'oxyde formant couche est la silice.

Cette encapsulation par de la silice peut être mise en oeuvre essentiellement selon deux variantes.

Dans le cas de la première variante, et qui est ici préférée à la deuxième variante exposée ci-après, le procédé consiste essentiellement à préparer la silice par hydrolyse d'un alkyl-silicate.
Plus précisément encore, on met en présence le support et un alkyl-silicate, on hydrolyse cet alkyl-silicate et on sépare le pigment formé et la phase liquide du milieu réactionnel.
On notera que le principe de cette voie est plus particulièrement décrit dans l'article de STÖBER et al., Journal of Colloïd and Interface Science, 26, pp. 62-69 (1968) et dont l'enseignement est incorporé à la présente description.
Généralement, il est préférable d'effectuer l'hydrolyse en présence d'une base qui joue alors le rôle de catalyseur.
On procède en formant un milieu réactionnel par mélange d'eau, d'alcool, du support qui est alors mis en suspension, et éventuellement d'une base, et en introduisant ensuite l'alkyl-silicate. La réaction est de préférence conduite sous agitation.
On peut utiliser comme base l'ammoniaque. Les alcools mis en oeuvre sont généralement des alcools aliphatiques, tel que par exemple du butanol. L'alkylsilicate est de préférence introduit avec un alcool.
Bien que la réaction puisse être conduite à température ambiante, on note toutefois une amélioration sensible de la qualité de la couche d'enrobage lorsque la température d'hydrolyse est choisie supérieure à cette température ambiante, en particulier comprise entre 40°C et 70°C.
Il est aussi possible de faire un pied de cuve à base d'alcool et d'alkyl-silicate, et d'y introduire ensuite l'eau ou un mélange eau-base.
Comme alkyl-silicate, on peut utiliser en particulier le silicate d'éthyle.
Après réaction et précipitation, le pigment obtenu est séparé du milieu réactionnel par toute technique connue en soi, en particulier par centrifugation ou par filtration, et est ensuite généralement lavé par de l'alcool, puis enfin séché.

Concernant la deuxième variante, le procédé consiste à mettre en présence, à mélanger et à faire réagir le support, un silicate et un acide, ce par quoi on précipite de la silice.
Selon un premier mode de réalisation de cette deuxième variante, l'acide et le silicate sont ajoutés simultanément dans le milieu réactionnel. Plus précisément, on forme tout d'abord une suspension aqueuse du support, et ensuite on ajoute simultanément l'acide et une solution aqueuse de silicate dans le milieu réactionnel.
Selon un mode de réalisation particulier, on peut alors introduire l'acide et le silicate en maintenant constant le pH du milieu réactionnel. Généralement, ce pH est fixé à une valeur comprise entre 8 et 9.
La température de réaction peut varier largement. En général, cette température est comprise entre 60 et 100°C.

Un deuxième mode (préféré au premier ci-dessus) de réalisation de cette deuxième variante du procédé selon l'invention consiste à former tout d'abord une suspension du support dans une solution aqueuse de silicate, et à ensuite introduire l'acide dans la suspension ainsi formée. Dans ce cas, le pH du milieu de précipitation est avantageusement fixé à une valeur supérieure à 7, et de préférence supérieure à 8, et ceci de manière à éviter une dissolution néfaste du support à base de sulfures de terres rares.
Dans cette deuxième variante, les conditions de température sont identiques à celles décrites dans la première.
En ce qui concerne le silicate, on utilise généralement un silicate alcalin et plus particulièrement les silicates de sodium, de potassium ou de lithium.
Pour les acides, on utilise habituellement un acide choisi dans le groupe comprenant les acides sulfurique, nitrique, chlorhydrique, carbonique. L'acide est généralement employé sous la forme d'une solution aqueuse.
La séparation entre le pigment d'une part et la phase liquide du milieu réactionnel d'autre part obtenus selon les procédés décrits ci-dessus se fait ensuite d'une manière connue en soi, par exemple par filtration. Le pigment séparé est ensuite séché.

Dans le cas où l'oxyde formant couche est l'alumine, plusieurs variantes peuvent aussi être envisagées.
Selon une première variante, on met en présence et on fait réagir le support, un aluminate et un acide, ce par quoi on précipite de l'alumine.
Selon un premier mode de réalisation de cette première variante, on pourra introduire simultanément l'aluminate et l'acide dans une suspension aqueuse du support. Dans ce cas, on peut opérer de manière à maintenir constant le pH du milieu réactionnel, ce pH étant de préférence supérieur à 7, et encore plus préférentiellement supérieur à 8.
Selon un deuxième mode de réalisation de cette première variante, on forme tout d'abord une suspension du support dans une solution d'aluminate, puis on introduit l'acide dans cette suspension. Là encore, on veillera à ce que le pH du milieu de réaction lors de l'introduction de l'acide reste de préférence supérieur à 7, ou même supérieur à 8.
D'une manière générale dans cette première variante, on utilise un aluminate alcalin. L'acide peut être, quant à lui, par exemple de l'acide chlorydrique ou de l'acide nitrique.
La seconde variante de préparation d'un pigment composite selon l'invention avec l'alumine comme oxyde formant couche consiste à mettre en présence, à mélanger et à faire réagir, le support, un sel d'aluminium et une base, ce par quoi l'on précipite de l'alumine.
Ainsi, on pourra par exemple partir d'un pied de cuve constitué par une suspension aqueuse du support, puis introduire de manière simultanée la base et le sel d'aluminium dans cette suspension.
La base utilisée est généralement la soude ou l'ammoniaque, et le sel d'aluminium pouvant être par exemple un halogénure d'aluminium tel que le chlorure d'aluminium, ou bien encore le nitrate d'aluminium.
Enfin, selon une troisième variante, on peut préparer les pigments composites avec l'alumine formant couche en obtenant ladite alumine par hydrolyse d'un alcoolate d'aluminium.
Cette variante est à rapprocher de celle décrite plus haut pour l'hydrolyse d'un alkyl-silicate.
On procède alors en mettant en présence le support et un alcoolate d'aluminium, on hydrolyse cet alcoolate et on sépare le pigment formé et la phase liquide du milieu réactionnel.
Tout ce qui a été décrit plus haut dans le cadre de l'hydrolyse d'un alkyl-silicate s'applique ici par analogie, notamment en ce qui concerne l'emploi d'une base et le mode d'introduction des réactifs.
On peut utiliser à titre d'alcoolates d'aluminium le méthylate, I'éthylate, l'isopropylate, le butylate d'aluminium, ces alcoolates étant sous forme liquide ou solide en dispersion ou en solution dans un solvant organique, par exemple le benzène ou l'alcool correspondant.

Dans le cas de la préparation d'un pigment composite comprenant le dioxyde de titane comme oxyde formant couche, on peut envisager différentes méthodes.
La première consiste à réaliser une précipitation de TiO₂ en introduisant dans une suspension aqueuse du support un sel de titane d'une part tel que TiCl₄, TiOCl₂ ou TiOSO₄, et une base d'autre part telle que de la soude ou de l'ammoniaque, l'introduction du sel et de la base se faisant de manière simultanée. On peut ensuite procéder à un mûrrissement du pigment.
Une deuxième méthode consiste à hydrolyser un titanate d'alkyle, par exemple le titanate d'isopropyle. Cette méthode est du même type que celle qui a été décrite plus haut pour l'hydrolyse d'un alkyl-silicate. On procède donc en mettant en présence le support et un titanate d'alkyle, puis on hydrolyse le titanate d'alkyle et on sépare le pigment formé et la phase liquide du milieu. Tout ce qui a été décrit plus haut à ce sujet concernant le mode d'introduction des réactifs s'applique ici.

Dans le cadre de la préparation de pigments composites comprenant l'oxyde de zirconium comme oxyde formant couche, les méthodes de préparation sont du même type que celles qui viennent d'être définies plus haut pour le dioxyde de titane, à savoir précipitation par réaction entre un sel de zirconium et une base ou hydrolyse d'un zirconate d'alkyle.

De même, dans le cadre de la préparation de pigments composites comprenant un ou des oxydes de terres rares à titre d'oxydes formant couche, on peut procéder soit par hydrolyse d'un alcoolate de terre rare, soit par précipitation en introduisant, et ceci de manière simultanée, dans une suspension aqueuse du support d'une part un sel de terre rare (nitrate ou chlorure) et une base d'autre part (soude, ammoniaque, par exemple).

Enfin, dans le cas du zircon formant couche autour du support, il est possible de procéder de la manière suivante: on co-hydrolyse dans une suspension aqueuse du support un alcoolate de zirconium d'une part et un alcoolate de silicium d'autre part, et ceci en présence de fluorure de sodium NaF, on récupère le pigment ainsi formé puis on cacine ce pigment de manière à transformer la couche d'enrobage précipitée en une phase de zircon, NaF jouant alors un rôle de fondant aidant à réaliser ladite transformation à la température la plus basse possible.

D'une manière générale, on notera qu'il est toujours possible de rester dans le cadre de la présente invention en préparant des pigments ayant soit plusieurs oxydes formant plusieurs couches succéssives, soit des mélanges d'oxydes ou oxydes mixtes formant une même couche.
En outre, on peut également mentioner la possibilité d'effectuer un ou des prétraitements du support (avant enrobage) dans le but d'améliorer encore certaines de ses caractéristiques ou propriétés au cours du traitement d'enrobage, ou même d'améliorer certaines des caractéristiques ou propriétés du pigment composite résultant.

La granulométrie des pigments composites récupérés est fine et régulière, et peut se situer entre 1 et 5 microns. Avec une telle granulométrie, les produits sont directement utilisables comme pigments, notamment pour matières plastiques. Comme indiqué précédemment dans la description, cette granulométrie est en relation directe avec la granulométrie initiale des particules de support mises en oeuvre dans le procédé d'enrobage. Cette dernière doit donc être choisie en fonction et en prévision de la granulométrie désirée pour le pigment composite final en vue d'une application particulière donnée.

Outre leurs excellentes couleurs intrinsèques (pigments fluorurés, encapsulés ou non) et leur stabilité chimique et/ou thermique améliorée (pigments fluorurés encapsulés), les pigments selon l'invention possèdent un très bon pouvoir de coloration et un très bon pouvoir opacifiant, et , de ce fait, conviennent parfaitement à la coloration de nombreux matériaux, tels que plastiques, peintures et autres. A cet égard, la polyvalence des pigments selon l'invention constitue l'un de leurs grands atouts.

Ainsi, et plus précisémment encore, ils peuvent être utilisés dans la coloration de matières plastiques qui peuvent être du type thermoplastiques ou thermodurcissables.
Comme résines thermoplastiques susceptibles d'être colorées selon l'invention, on peut citer, à titre purement illustratif, le chlorure de polyvinyle, l'alcool polyvinylique, le polystyrène, les copolymères styrène-butadiène, styrèneacrylonitrile, acrylonitrile-butadiène-styrène (A.B.S.), les polymères acryliques notamment le polyméthacrylate de méthyle, les polyoléfines telles que le polyéthylène, le polypropylène, le polybutène, le polyméthylpentène, les dérivés cellulosiques tels que par exemple l'acétate de cellulose, I'acéto-butyrate de cellulose, I'éthylcéllulose, les polyamides dont le polyamide 6-6.

Concernant les résines thermodurcissables pour lesquelles les pigments selon l'invention conviennent également, on peut citer, par exemple, les phénoplastes, les aminoplastes notamment les copolymères urée-formol, mélamine-formol, les résines époxy et les polyesters thermodurcissables.

On peut également mettre en oeuvre les pigments de l'invention dans des polymères spéciaux tels que des polymères fluorés en particulier le polytétrafluoréthylène (P.T.F.E.), les polycarbonates, les élastomères silicones, les polyimides.
Dans cette application spécifique pour la coloration des plastiques, on peut mettre en oeuvre les pigments de l'invention directement sous forme de poudres. On peut également, de préférence, les mettre en oeuvre sous une forme pré-dispersée, par exemple en prémélange avec une partie de la résine, sous forme d'un concentré pâte ou d'un liquide ce qui permet de les introduire à n'importe quel stade de la fabrication de la résine. Ce dernier point constitue un avantage particulièrement important des pigments selon l'invention.

Ainsi, les pigments selon l'invention peuvent être incorporés dans des matières plastiques telles que celles mentionnées ci-avant dans une proportion pondérale allant généralement soit de 0,01 à 5% (ramenée au produit final) soit de 40 à 70% dans le cas d'un concentré.

Les pigments de l'invention peuvent être également utilisés dans le domaine des peintures et lasures et plus particulièrement dans les résines suivantes : résines alkydes dont la plus courante est dénommée glycérophtalique; les résines modifiées à l'huile longue ou courte; les résines acryliques dérivées des esters de l'acide acrylique (méthylique ou éthylique) et méthacrylique éventuellement copolymérisés avec l'acrylate d'éthyle, d'éthyl-2 hexyle ou de butyle; les résines vinyliques comme par exemple l'acétate de polyvinyle, le chlorure de polyvinyle, le butyralpolyvinylique, le formalpolyvinylique, et les copolymères chlorure de vinyle et acétate de vinyle ou chlorure de vinylidène; les résines aminoplastes ou phénoliques le plus souvent modifiées; les résines polyesters; les résines polyuréthanes; les résines époxy; les résines silicones.
Généralement, les pigments sont mis en oeuvre à raison de 5 à 30% en poids de la peinture, et de 0,1 à 5% en poids du lasure.

Enfin, les pigments selon l'invention sont également susceptibles de convenir pour des applications dans l'industrie du caoutchouc, notamment dans les revêtements pour sols, dans l'industrie du papier et des encres d'imprimerie, dans le domaine de la cosmétique, ainsi que nombreuses autres utilisations comme par exemple, et non limitativement, le finissage des cuirs et les revêtements stratifiés pour cuisines et autres plans de travail, les céramiques.

En ce qui concerne plus particulièrement la cosmétique, les pigments de l'invention peuvent être utilisés dans les vernis à ongles et dans les fards tels que rouges à lèvres, fards secs, fards gras ou fonds de teint.

Ils peuvent donc être mis en oeuvre dans les vernis à ongles qui contiennent généralement:
- un agent filmogène à base de nitrocellulose,
- une résine, résine dammer naturelle ou résine synthétique du type formaldéhyde -sulfamide, résine polystyrène, résine polyvinyique etc...
- un plastifiant par exemple, le phtalate de diéthyle, le phtalate de dibutyle, le phtalate de dioctyle, le tricrésylphosphate, le stéarate de n-butyle, le diacétate de résorcine ou leur mélange,
- un dissolvant tel que l'alcool éthylique, isopropylique, butylique, isobutylique, I'acétate d'éthyle, I'acétate de butyle ou le plus souvent le mélange de ces solvants,
- un diluant, notamment le toluène ou le xylène,
- éventuellement d'autres additifs, parfum ou produit nacrant (flocons de mica enrobés d'oxychlorure de bismuth ou de bioxyde de titane).

On donne ci-après un exemple de composition-type :
- de 10 à 15% en poids de nitrocellulose,
- de 10 à 15% en poids de résine,
- de 3 à 5% en poids de plastifiant(s),
- de 3 à 5% en poids de pigment(s),
- q.s.p. 100% en poids de solvant(s).

Généralement, les pigments sont broyés dans une masse plastique constituée de nitrocellulose et de plastifiant(s) qui est ensuite mise en solution dans le(s) solvant(s).

Une autre application des pigments de l'invention est celle des rouges à lèvres.

Les pigments sont mis en oeuvre le plus souvent à raison d'une concentration pondérale de 5 à 15% exprimée par rapport à la formulation totale qui renferme :
- un excipient formé d'un mélange de divers corps pour assurer la consistance : cire d'abeille, cire de carnauba, ozocérites, paraffine, cires synthétiques ou leur mélange et d'un excipient mou permettant d'ajuster la consistance tel que le beurre de cacao, la vaseline, les huiles blanches hydrogénées par exemple, l'huile de palme, d'arachide ou de ricin,
- divers additifs notamment un parfum ou arome et le myristate d'isopropyle ou le palmitate d'isopropyle qui donne du glissant,
- un solvant intermédiaire pour mettre en suspension le pigment dans la phase lipophyle qui peut être l'huile de ricin ou un glycol comme le polyoxyéthylèneglycol 400 ou des esters d'acides gras : monoricinoléate de propylène glycol, myristate d'isopropyle, palmitate d'isopropyle, stéarate de butyle.

Les fards à yeux et les fards à joues peuvent se présenter sous forme de fards secs ou de fards gras. La teneur en pigments dans de tels fards peut varier dans de larges limites de 5 à 20%.

Les fards secs sont des poudres (talc, carbonate de magnésium, stéarate de zinc) qui sont chargées en pigments et agglomérées soit avec de la méthylcellulose, soit avec des stéarates.

On donne, à titre d'exemple la composition d'un fard à paupières :
- silicate d'aluminium et de magnésium (Veegum F): 7% en poids
- talc : 50% en poids
- oxyde de zinc : 4% en poids
- stéarate de zinc : 11% en poids
- kaolin : 10% en poids
- pigment : 18% en poids

Les pigments de l'invention peuvent également être employés dans les formulations de fonds de teint.

Les fonds de teint se présentent sous forme d'émulsion en général du type huile dans l'eau.

La phase lipophyle comprend le plus souvent :
- un composant huileux tel que l'huile de vaseline, des esters d'acides gras et d'alcools éventuellement gras, par exemple, I'oléate d'oléyle, I'oléate de décyle, le stéarate d'octyle, l'adipate de di-n-butyle, le myristate d'isopropyle, le palitate d'isopropyle, le stéarate d'isopropyle, les esters d'acides caprique et caprylique d'alcools gras saturés ayant de 12 à 18 atomes de carbone, une huile de silicone ou leur mélange entre eux,
- un agent émulsifiant du type anionique et/ou non-ionique et plus précisément les sels d'acides gras, stéarate de sodium, de potassium ou d'ammonium, palmitate de sodium : les esters de sorbitan et d'acides gras tels que, par exemple, l'acide laurique, l'acide palmitique, l'acide stéarique; les esters polyoxyéthylénés de sorbitan et d'acides gras contenant de 4 à 20 moles d'oxyde d'éthylène par mole d'ester : les alcools gras polyoxyéthylénés contenant de 2 à 23 moles d'oxyde d'éthylène par mole d'alcool, ledit alcool pouvant être notamment l'alcool laurique, l'alcool cétylique, l'alcool stéarylique, l'alcool oléylique; le mono-et distéarate de glycérol, le mono-et dioléate de glycérol; les acides gras poxyloxyéthylénés et en particulier le stéarate polyoxyéthyléné contenant de 18 à 100 moles d'oxyde d'éthylène par mole d'acide,
- un agent permettant d'ajuster la consistance qui peut être un alcool gras ou un acide gras et plus précisément l'alcool cétylique, l'alcool stéarylique, l'acide stéarique.

Quant à la phase hydrophile, elle est constituée d'eau, de préférence distillée et de divers additifs, notamment :
- un agent humectant qui peut être, par exemple, le propylèneglycol, le glycérol, le sorbitol,
- un agent conservateur et plus particulièrement l'ophénylphénol et les acides suivants, leurs sels (Na, K, NH₄) ou leurs esters ayant de 1 à 4 atomes de carbone : l'acide benzoïque, l'acide salicylique, l'acide sorbique, l'acide p-hydroxybenzoïque,
- un agent de stabilité notamment les dérivés cellulosiques dont la carboxyméthylcellulose et la gomme Xanthane.

Une illustration d'une formulation pour fonds de teint est donnée ci-après :--- phase lipophyle:
- huile de vaseline : 15% en poids
- mono-et distéarate de glycérol : 4% en poids
- alcool cétylique : 1% en poids
- phase hydrophyle :
- eau distillée q.s.p. : 100% en poids
- propylène glycol : 3% en poids
- para-oxybenzoate de méthyle : 0,05% en poids
- para-oxybenzoate de propyle : 0,1% en poids
- pigment coloré : 1 à 10% en poids
- oxyde de titane : 3% en poids

La préparation des formulations de fonds de teint est conduite en dispersant d'abord le pigment dans la phase lipophile maintenue vers 60-80°C puis la phase hydrophyle maintenue à une des températures comprises dans l'intervalle précité est ajoutée sous agitation et lentement dans la phase lipophile.

Dans l'exposé qui précède, il est donné des exemples de formulations destinées à la cosmétique dans lesquelles peuvent convenir les pigments de l'invention et il va sans dire que ces exemples de même que les différents composants cités ne présentent aucun caractère limitatif et ne sont donnés qu'à titre illustratif.

Des exemples concrêts illustrant l'invention vont maintenant être donnés.

### Exemple 1

Un sesquisulfure de cérium Ce₂S₃ γ cubique dopé au sodium a été préparé selon l'enseignement de la demande de brevet FR 91/14988, en calcinant à 1050°C dans un four tubulaire étanche à l'air, préalablement purgé, et dans lequel on réalise un balayage continu d'argon chargé à 10% d'hydrogène, un mélange d'oxalate de cérium, de carbonate de sodium anhydre et de soufre élémentaire qui a été placé dans une nacelle consommable en carbone graphite, le rapport molaire initial Na/Ce dans le mélange ayant été fixé à 0,2.

Le produit récupéré après calcination est ensuite lavé une fois avec de l'eau permutée.

Le produit obtenu présente alors une couleur rouge très intense. L'analyse en diffraction X montre que l'on a obtenu la seule phase Ce₂S₃ γ cubique, avec un paramètre de maille égal à 8,637 A. La teneur en sodium du produit est de 2,25% en poids (Na/Ce₂S₃). Il se présente sous la forme d'une poudre ayant une granulométrie moyenne (Φ₅₀) de 4,33 µm (mesure par granulomètre laser CILAS).

Les coordonnées chromatiques de ce produit témoin sont les suivantes:
L*=47
a*=49
b*=33
10 g du produit témoin préparé précédemment sont alors introduits dans 100 ml d'une solution de fluorure d'ammonium à 10 g/l. Puis, par ajout d'une solution d'ammoniaque NH₄OH, on porte le pH du mélange ainsi obtenu à une valeur légérement basique, de l'ordre de 8, (le but de cette opération est d'éviter toute solubilisation intempestive du pigment dûe à un milieu trop acide) et on laisse alors le tout sous agitation pendant une heure.
Le produit est ensuite filtré, puis séché dans un dessicateur sous vide.

Les nouvelles caractéristiques chromatiques du produit ainsi obtenu sont alors les suivantes:
L*=47
a*=55
b*=43

Sa granulométrie moyenne (Φ₅₀) est de 3,67 µm.

Ce produit contient 2,9% en poids de fluor.

En outre, les analyses en diffraction X permettent de détecter, entre autres, la présence de la phase CeF₃.

### Exemple 2

On procède comme à l'exemple 1 ci-dessus, à cette différence près que le produit témoin de sesquisulfure de cérium Ce₂S₃ γ cubique dopé sodium a été obtenu en mettant en oeuvre un mélange initial dans lequel le rapport molaire Na/Ce a été fixé à 0,25.

10 g de ce produit témoin ont ensuite été fluorurés dans les mêmes conditions que celles de l'exemple 1.

Les caractéristiques chromatiques des produits obtenus sont données dans le tableau ci-dessous.

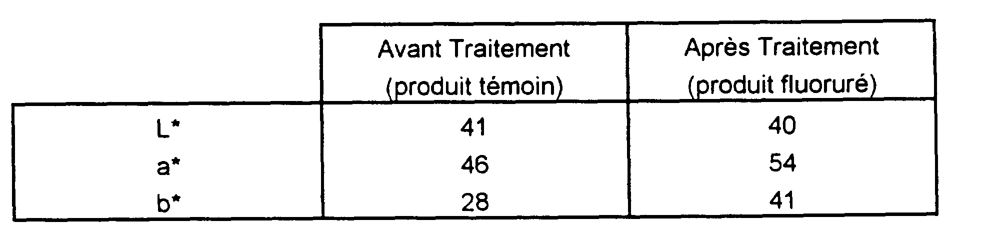

### Exemple 3

Un sesquisulfure de cérium Ce₂S₃ γ cubique dopé au sodium a été préparé en calcinant à 800°C dans une atmosphère d'argon contenant du sulfure de carbone (CS₂) à raison de 0,3 bar en pression partielle, un mélange d'oxyde de cérium et de carbonate de sodium anhydre, le rapport molaire initial Na/Ce dans ledit mélange ayant été fixé à 0,15.

10 g de ce produit témoin ont été ensuite fluorurés dans les mêmes conditions que celles de l'exemple 1.

Les caractéristiques des produits obtenus sont données dans le tableau ci-dessous.

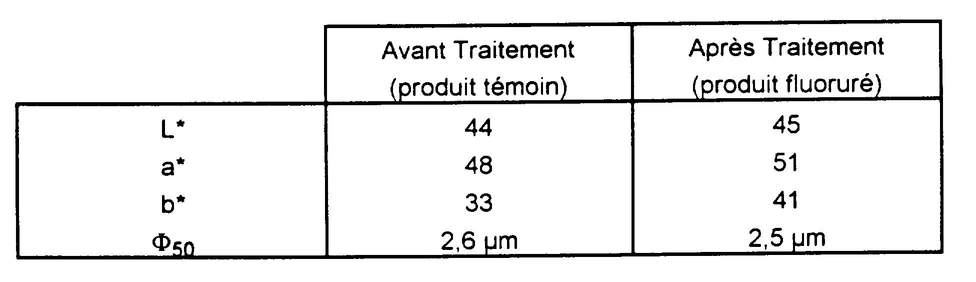

### Exemple 4

A l'inverse des exemples précédents (fluoruration par voie humide), cet exemple illustre l'invention dans le cadre d'un traitement de fluoruration par voie gaz, et ceci au moyen d'un plasma de fluorure d'azote NF₃.

Le principe du traitement est le suivant :
Le gaz NF₃ est introduit en continu dans une enceinte réactionnelle maintenue à 80°C puis dissocié entre deux électrodes, les espèces ainsi formées diffusant alors dans ladite enceinte pour venir réagir avec le pigment qui a été préalablement placé dans cette dernière. Plusieurs paramètres permettent de contrôler la cinétique et l'avancement de la réaction de fluoruration: la puissance du générateur de radiofréquences permettant la dissociation du gaz (P), le débit de gaz injecté (d), la pression totale dans l'enceinte (p) et la durée du traitement (t). Dans le cas présent, l'avancement de la réaction est suivi expérimentalement par la prise de masse du pigment.

On a ainsi traité, selon la technique ci-dessus exposée, 0,1 g du pigment témoin préparé à l'exemple 2. Les paramètres réactionnels retenus pour conduire ce traitement étaient les suivants : P = 50 W; d = 8 cm³/s ; p = 800 mTorr ; tₘₐₓ = 5 mn.

Les caractéristiques des produits obtenus sont données dans le tableau ci-dessous.

| **Prise de masse (%)** | **L*** | **a*** | **b*** |
|---|---|---|---|
| 0 (avant traitement) | 41 | 46 | 28 |
| 1,2 | 39 | 51 | 40 |
| 1,6 | 39 | 53 | 47 |
| 2,1 | 39 | 55 | 46 |

Les analyses Auger et ESCA montrent, pour les produits traités, une surface enrichie en fluor et en cérium. Les analyses en diffraction X révèlent, quant à elles, la présence de composés fluorés du type CeF₃ ou CeSF.

### Exemple 5

Cet exemple illustre un autre un autre mode de réalisation d'une fluoruration par voie gaz. On procède ici au traitement du pigment au moyen d'un mélange gazeux constitué de fluor (F₂) dilué dans un gaz neutre, ici de l'azote (N₂).

Le traitement de fuoruration a été éffectué en régime statique dans un réacteur en nickel contenant, sous une pression totale de 1 bar, de l'azote dans lequel était dilué du fluor à raison de 10% en volume. La température du traitement était de 80°C. La durée maximale de traitement a été fixée à 2 heures. Comme à l'exemple précédent, l'avancement de la réaction de fluoruration est ici suivie expérimentalement par la prise de masse du pigment.

Le pigment ici traité est le pigment témoin préparé à l'exemple 2.

Les caractéristiques des produits obtenus sont données dans le tableau ci-dessous.

| **Prise de masse (%)** | **L*** | **a*** | **b*** |
|---|---|---|---|
| 0 (avant traitement) | 41 | 46 | 28 |
| 1,3 | 39 | 54 | 51 |
| 1,7 | 38 | 59 | 50 |
| 2,5 | 40 | 58 | 59 |

Les mêmes observations que celles faites à l'exemple 4 précédent, concernant la nature chimique de la surface des produits traités, ont pu être faites.

### EXEMPLE 6

Cet exemple illustre l'utilisation d'un produit de l'invention dans la préparation d'un rouge à lèvres.

Les éléments constitutifs du rouge à lèvres sont donnés ci-dessous:

| **CONSTITUANTS** | **% EN POIDS** |
|---|---|
| **A** | |
| Silbione huile 70641 V 2OO | 40,5 |

| **B** | |
|---|---|
| Cire d'abeille | 7,5 |
| Cire de carnauba | 3,5 |
| Ozocérite | 3,5 |
| Paraffine | 10,0 |
| Huile de vaseline | q.s.p. 100 |
| Triglycéride d'acide caprique/caprylique (*) | 20,0 |

| **C** | |
|---|---|
| Pigments selon exemple 3 | q.s. |
| Oxyde de titane | q.s. |

| | |
|---|---|
| (*) MYRITOL 318 de HENKEL | |

Les éléments du mélange B sont fondus à une température de 85 ± 2°C, puis maintenus au bain thermostaté régulé à 60 ± 2°C.

Le pigment et l'oxyde de titane sont malaxés avec l'huile SILBIONE; ce mélange A-C est placé au bain thermostaté (60 ± 2°C).

Le mélange B est ensuite ajouté.

L'ensemble est coulé dans un moule siliconé.

## Revendications

1. Procédé pour améliorer la coloration d'un pigment à base de sesquisulfure(s) de terre(s) rare(s), caractérisé en ce qu'il consiste à soumettre ledit pigment à un traitement de fluoruration.

2. Procédé selon la revendication 1, caractérisé en ce que ledit traitement de fluoruration se limite essentiellement à la surface dudit pigment.

3. Procédé selon l'une quelconque des revendications précédentes, caractérisé en ce que ladite fluoruration est mise en oeuvre au moyen d'un agent fluorurant choisi, seul ou en mélange, parmi le fluor, les fluorures d'halogènes, le fluorure d'ammonium, les fluorures de gaz rares, le fluorure d'azote, le fluorure de bore, le tétrafluorométhane et l'acide fluorhydrique

4. Procédé selon la revendication 3, caractérisé en ce que l'on utilise du fluor ou du fluorure d'ammonium.

5. Procédé selon l'une quelconque des revendications précédentes, caractérisé en ce que ladite fluoruration se fait par voie liquide ou par voie gaz.

6. Procédé selon l'une quelconque des revendications précédentes, caractérisé en ce que la quantité de fluor introduite dans ledit pigment par ledit traitement n'excède pas 10% en poids par rapport au pigment.

7. Procédé selon la revendication 6, caractérisé en ce que ladite quantité n'excède pas 5% en poids.

8. Procédé selon l'une quelconque des revendications précédentes, caractérisé en ce que ledit pigment à base de sesquisufure(s) de terre(s) rare(s) contient en outre au moins un élément alcalin et/ou alcalino-terreux (élément dopant) dont une partie au moins est incluse dans le réseau cristallin du sesquisulfure.

9. Procédé selon la revendication 8, caractérisé en ce que ledit élément dopant est choisi, seul ou en mélange, parmi les éléments alcalins.

10. Procédé selon la revendication 9, caractérisé en ce que ledit élément dopant est le sodium.

11. Procédé selon l'une quelconque des revendications précédentes, caractérisé en ce que ledit sesquisulfure de terre(s) rare(s) est le sesquisulfure de cérium Ce₂S₃ γ cubique, de préférence dopé par du sodium.

12. Procédé selon l'une quelconque des revendications précédentes, caractérisé en ce que l'on procède ensuite à une encapsulation du pigment fluoruré ainsi obtenu par une couche enrobante à base d'au moins un oxyde transparent.

13. Pigments colorés à base de sesquisulfure(s) de terre(s) rare(s), caractérisés par le fait qu'ils contiennent des atomes de fluor.

14. Pigments selon la revendication 13, caractérisés en ce que lesdits atomes de fluor sont distribués selon un gradient de concentration décroissant de la surface au coeur dudit sesquisulfure.

15. Pigments selon l'une quelconque des revendications 13 ou 14, caractérisés en ce que lesdits atomes de fluor sont majoritairement répartis à la périphérie externe des particules constituant ledit sesquisulfure.

16. Pigments selon l'une quelconque des revendications 13 à 15, caractérisés en ce que le pourcentage en poids desdits atomes de fluor par rapport audit sesquisulfure n'excède pas 10%.

17. Pigments selon la revendication 16, caractérisés en ce que ledit pourcentage n'excède pas 5%.

18. Pigments selon l'une quelconque des revendications 13 à 17, caractérisés en ce que lesdits atomes de fluor sont présents dans lesdits pigments sous la forme de composés fluorés ou sulfofluorés.

19. Pigments selon la revendication 18, caractérisés en ce que lesdits composés sont des fluorures et/ou des sulfofluorures de terres rares.

20. Pigments selon l'une quelconque des revendications 13 à 19, caractérisés en ce qu'ils contiennent en outre au moins un élément alcalin et/ou alcalino-terreux (élément dopant) dont une partie au moins est incluse dans le réseau cristallin dudit sesquisulfure.

21. Pigments selon la revendication 20, caractérisés en ce que ledit élément dopant est essentiellement présent sous une forme incluse dans le réseau cristallin dudit sesquisulfure.

22. Pigments selon l'une quelconque des revendications 20 ou 21, caractérisés en ce que ledit élément dopant est présent dans les lacunes cationiques du réseau cristallin dudit sesquisulfure.

23. Pigments selon l'une quelconque des revendications 20 à 22, caractérisés en ce que la quantité molaire en élément dopant représente au moins 0,1% de la quantité molaire en terre(s) rare(s).

24. Pigments selon la revendication 23, caractérisés en ce que la quantité molaire en élément dopant est comprise entre 5 et 50% de la quantiré molaire en terre(s) rare(s).

25. Pigments selon l'une quelconque des revendications 20 à 24, caractérisés en ce que ledit élément dopant est choisi, seul ou en mélange, parmi les éléments alcalins.

26. Pigments selon la revendication 25, caractérisés en ce que ledit élément dopant est le sodium.

27. Pigments selon l'une quelconque des revendications 13 à 26, caractérisés en ce que ledit sesquisulfure de terre(s) rare(s) est le sesquisulfure de cérium Ce₂S₃ γ cubique.

28. Pigments selon la revendication 27, caractérisés en ce qu'ils présentent une coordonnée chromatique L* au moins égale à 30.

29. Pigments selon la revendication 28, caractérisés en ce que ladite coordonnée chromatique L* est comprise entre 30 et 60.

30. Pigments selon l'une quelconque des revendications 27 à 29, caractérisés en ce qu'ils présentent une coordonnée chromatique a* au moins égale à 30.

31. Pigments selon la revendication 30, caractérisés en ce que ladite coordonnée chromatique a* est comprise entre 35 et 65.

32. Pigments selon l'une quelconque des revendications 27 à 31, caractérisés en ce qu'ils présentent une coordonnée chromatique b* comprise entre 0 et 40.

33. Pigments selon l'une quelconque des revendications 13 à 32, caractérisés en ce qu'ils présentent une granulométrie moyenne comprise entre 1 et 5 µm.

34. Pigments selon l'une quelconque des revendications 13 à 33, caractérisés en ce qu'ils sont constitués de sesquisulfure de cérium Ce₂S₃ γ cubique dopé par du sodium.

35. Pigments selon l'une quelconque des revendications 13 à 34, caractérisés par le fait qu'ils comprennent en outre une couche enrobante à base d'au moins un oxyde transparent déposée à leur surface.

36. Pigments selon la revendication 35, caractérisés en ce que ledit oxyde transparent est choisi, seul ou en mélange, dans le groupe constitué par la silice, l'alumine, la zircone, le zircon, l'oxyde de titane et les oxydes de terres rares.

37. Pigments selon l'une des revendications 35 ou 36, caractérisés en ce que ladite couche est à base de silice.

38. Pigments selon la revendication 37, caractérisés en ce que ladite couche est uniquement constituée de silice.

39. Pigment selon l'une des revendications 35 à 38, caractérisés en ce qu'ils sont constitués d'un support en sesquisulfure de cérium Ce₂S₃ γ cubique dopé par du sodium et contenant des atomes de fluor, lequel support est enrobé par une couche homogène de silice transparente.

40. Pigments colorés, caractérisés par le fait qu'ils sont obtenus par la mise en oeuvre d'un procédé tel que défini à l'une quelconque des revendications 1 à 12.

41. Utilisation des pigments colorés définis à l'une quelconque des revendications 13 à 40, dans des matières plastiques, des peintures, des lasures, des caoutchoucs, des céramiques, des glaçures, des papiers, des encres, des produits cosmétiques, des teintures et des revêtements stratifiés.

42. Compositions de matière colorées notamment du type plastiques, peintures, lasures, caoutchoucs, céramiques, glaçures, papiers, encres, produits cosmétiques, teintures et revêtement stratifiés, caractérisées en ce qu'elles comprennent des pigments colorés tels que définis à l'une quelconque des revendications 13 à 40.

## Patentansprüche

1. Verfahren zum Verbessern der Färbung eines Pigments auf Basis von Sesquisulfid(en) von Seltenerdelement(en),
dadurch gekennzeichnet, daß es darauf beruht, das Pigment einer Fluoridierungsbehandlung zu unterziehen.

2. Verfahren nach Anspruch 1,
dadurch gekennzeichnet, daß die Fluoridierungsbehandlung sich im wesentlichen auf die Oberfläche des Pigments beschränkt.

3. Verfahren nach einem der vorangegangenen Ansprüche,
dadurch gekennzeichnet, daß die Fluoridierung mittels eines Fluoridierungsmittels bewirkt wird, das als Einzelverbindung oder in Form einer Mischung aus Fluor, den Halogenfluoriden, Ammoniumfluorid, den Edelgasfluoriden, Stickstofffluorid, Borfluorid, Tetrafluormethan und Fluorwasserstoffsäure ausgewählt ist.

4. Verfahren nach Anspruch 3,
dadurch gekennzeichnet, daß Fluor oder Ammoniumfluorid verwendet wird.

5. Verfahren nach einem der vorangegangenen Ansprüche,
dadurch gekennzeichnet, daß die Fluoridierung durch Flüssigkeit oder Gas erfolgt.

6. Verfahren nach einem der vorangegangenen Ansprüche,
dadurch gekennzeichnet, daß die Menge an Fluor, die in das Pigment durch die Behandlung eingeführt wird, 10 Gew.-% bezogen auf das Pigment nicht übersteigt.

7. Verfahren nach Anspruch 6,
dadurch gekennzeichnet, daß die Menge 5 Gew.-% nicht übersteigt.

8. Verfahren nach einem der vorangegangenen Ansprüche,
dadurch gekennzeichnet, daß das Pigment auf Basis von Sesquisulfid(en) von Seltenerdelement(en) darüberhinaus mindestens ein Alkalielement und/oder Erdalkalielement (Dotierungselement) enthält, von dem mindestens ein Teil in dem Kristallgitter des Sesquisulfid eingeschlossen ist.

9. Verfahren nach Anspruch 8,
dadurch gekennzeichnet, daß das Dotierungselement als Einzelverbindung oder in Form einer Mischung aus den Alkalielementen ausgewählt wird.

10. Verfahren nach Anspruch 9,
dadurch gekennzeichnet, daß das Dotierungselement Natrium ist.

11. Verfahren nach einem der vorangegangenen Ansprüche,
dadurch gekennzeichnet, daß das Sesquisulfid von Seltenerdelement(en) das kubische γ-Cersesquisulfid Ce₂S₃, vorzugsweise mit Natrium dotiert, ist.

12. Verfahren nach einem der vorangegangenen Ansprüche,
dadurch gekennzeichnet, daß man sodann eine Einkapselung des so erhaltenen fluoridierten Pigments durch einen umhüllenden Überzug auf Basis von mindestens einem transparenten Oxid vornimmt.

13. Gefärbte Pigmente auf Basis von Sesquisulfid(en) von Seltenerdelement(en), dadurch gekennzeichnet, daß sie Fluoratome enthalten.

14. Pigmente nach Anspruch 13,
dadurch gekennzeichnet, daß die Fluoratome entsprechend einem Konzentrationsgradienten verteilt sind, der von der Oberfläche zum Kern des Sesquisulfids abnimmt.

15. Pigment nach einem der Ansprüche 13 oder 14,
dadurch gekennzeichnet, daß die Fluoratome in der Mehrzahl an der äußeren Peripherie der Teilchen, die das Sesquisulfid darstellen, verteilt sind.

16. Pigmente nach einem der Ansprüche 13 bis 15,
dadurch gekennzeichnet, daß der Gewichtsprozentsatz der Fluoratome bezogen auf das Sesquisulfid 10 % nicht übersteigt.

17. Pigmente nach Anspruch 16,
dadurch gekennzeichnet, daß der Prozentsatz 5 % nicht übersteigt.

18. Pigmente nach einem der Ansprüche 13 bis 17,
dadurch gekennzeichnet, daß die Fluoratome in den Pigmenten in Form von Fluor- oder Sulfofluorverbindungen vorliegen.

19. Pigmente nach Anspruch 18,
dadurch gekennzeichnet, daß die Verbindungen Fluoride und/oder Sulfofluoride von Seltenerdelementen sind.

20. Pigmente nach einem der Ansprüche 13 bis 19,
dadurch gekennzeichnet, daß sie darüberhinaus mindestens ein Alkalielement und/oder Erdalkalielement (Dotierungselement) enthalten, von dem mindestens ein Teil in dem Kristallgitter des Sesquisulfids eingeschlossen ist.

21. Pigmente nach Anspruch 20,
dadurch gekennzeichnet, daß das Dotierungselement im wesentlichen in einer in dem Kristallgitter des Sesquisulfids eingeschlossenen Form vorliegt.

22. Pigmente nach einem der Ansprüche 20 oder 21,
dadurch gekennzeichnet, daß das Dotierungselement in den kationischen Gitterfehlstellen des Kristallgitters des Sesquisulfids vorliegt.

23. Pigmente nach einem der Ansprüche 20 bis 22,
dadurch gekennzeichnet, daß die molare Menge an Dotierungselement mindestens 0,1 % der molaren Menge an Seltenerdelement(en) darstellt.

24. Pigmente nach Anspruch 23,
dadurch gekennzeichnet, daß die molare Menge an Dotierungselement zwischen 5 und 50 % der molaren Menge an Seltenerdelement(en) beträgt.

25. Pigmente nach einem der Ansprüche 20 bis 24,
dadurch gekennzeichnet, daß das Dotierungselement als Einzelverbindung oder in Form einer Mischung aus den Alkalielementen ausgewählt wird.

26. Pigmente nach Anspruch 25,
dadurch gekennzeichnet, daß das Dotierungselement Natrium ist.

27. Pigmente nach einem der Ansprüche 13 bis 26,
dadurch gekennzeichnet, daß das Sesquisulfid von Seltenerdelement(en) das kubische γ-Cersesquisulfid Ce₂S₃ ist.

28. Pigmente nach Anspruch 27,
dadurch gekennzeichnet, daß sie eine Farbwertkoordinate L* von mindestens gleich 30 aufweisen.

29. Pigmente nach Anspruch 28,
dadurch gekennzeichnet, daß die Farbwertkoordinate L* zwischen 30 und 60 liegt.

30. Pigmente nach einem der Ansprüche 27 bis 29,
dadurch gekennzeichnet, daß sie eine Farbwertkoordinate a* von mindestens gleich 30 aufweisen.

31. Pigmente nach Anspruch 30,
dadurch gekennzeichnet, daß die Farbwertkoordinate a* zwischen 35 und 65 liegt.

32. Pigmente nach einem der Ansprüche 27 bis 31,
dadurch gekennzeichnet, daß sie eine Farbwertkoordinate b* zwischen 0 und 40 aufweisen.

33. Pigmente nach einem der Ansprüche 13 bis 32,
dadurch gekennzeichnet, daß sie eine mittlere Komklassierung zwischen 1 und 5 µm aufweisen.

34. Pigmente nach einem der Ansprüche 13 bis 33,
dadurch gekennzeichnet, daß sie aus mit Natrium dotiertem kubischem γ-Cersesquisulfid Ce₂S₃ aufgebaut sind.

35. Pigmente nach einem der Ansprüche 13 bis 34,
dadurch gekennzeichnet, daß sie darüberhinaus einen auf ihrer Oberfläche abgeschiedenen, umhüllenden Überzug auf Basis von mindestens einem transparenten Oxid umfassen.

36. Pigmente nach Anspruch 35,
dadurch gekennzeichnet, daß das transparente Oxid als Einzelverbindung oder in Form einer Mischung aus der Gruppe, gebildet aus Kieselerde, Aluminiumoxid, Zirkonoxid, Zirkon, Titanoxid und den Seltenerdoxiden, ausgewählt wird.

37. Pigmente nach Anspruch 35 oder 36,
dadurch gekennzeichnet, daß das Grundmaterial des Überzugs Kieselerde ist.

38. Pigmente nach Anspruch 37,
dadurch gekennzeichnet, daß der Überzug ausschließlich aus Kieselerde gebildet ist.

39. Pigmente nach einem der Ansprüche 35 bis 38,
dadurch gekennzeichnet, daß sie aus einem Träger von mit Natrium dotiertem, kubischem γ-Cersesquisulfid Ce₂S₃, das Fluoratome enthält, gebildet sind, wobei der Träger mit einem homogenen Überzug von transparenter Kieselerde umhüllt ist.

40. Gefärbte Pigmente,
dadurch gekennzeichnet, daß sie durch Ausführen eines Verfahrens, wie es in einem der Ansprüche 1 bis 12 definiert ist, erhalten werden.

41. Verwendung der in einem der Ansprüche 13 bis 40 definierten gefärbten Pigmente in Kunststoffmaterialien, Anstrichmitteln, Lasuren, Gummis, Keramiken, Glasuren, Papieren, Tinten, kosmetischen Produkten, Beizen und Schichtüberzügen.

42. Gefärbte Stoffzusammensetzungen insbesondere vom Typ Kunststoffmaterialien, Anstrichmittel, Lasuren, Gummis, Keramiken, Glasuren, Papieren, Tinten, kosmetische Produkte, Beizen und Schichtüberzüge,
dadurch gekennzeichnet, daß sie gefärbte Pigmente, wie sie in einem der Ansprüche 13 bis 40 definiert sind, umfassen.

## Claims

1. Process for improving the colouring of a pigment based on rare-earth metal sesquisulphide(s), characterized in that it consists in subjecting the said pigment to a fluoridation treatment.

2. Process according to claim 1, characterized in that the said fluoridation treatment is restricted essentially to the surface of the said pigment.

3. Process according to either of the preceding claims, characterized in that the said fluoridation is implemented by means of a fluoridating agent chosen, alone or as a mixture, from fluorine, halogen fluorides, ammonium fluoride, rare gas fluorides, nitrogen fluoride, boron fluoride, tetrafluoromethane and hydrofluoric acid.

4. Process according to claim 3, characterized in that fluorine or ammonium fluoride is used.

5. Process according to any one of the preceding claims, characterized in that the said fluoridation is carried out by a liquid route or by a gas route.

6. Process according to any one of the preceding claims, characterized in that the amount of fluorine introduced into the said pigment by the said treatment does not exceed 10% by weight with respect to the pigment.

7. Process according to claim 6, characterized in that the said amount does not exceed 5% by weight.

8. Process according to any one of the preceding claims, characterized in that the said pigment based on rare-earth metal sesquisulphide(s) additionally contains at least one alkali metal and/or alkaline-earth metal element (doping element), at least part of which is occluded in the crystal lattice of the sesquisulphide.

9. Process according to claim 8, characterized in that the said doping element is chosen, alone or as a mixture, from alkali metal elements.

10. Process according to claim 9, characterized in that the said doping element is sodium.

11. Process according to any one of the preceding claims, characterized in that the said rare-earth metal sesquisulphide is γ cubic cerium sesquisulphide Ce₂S₃, preferably doped with sodium.

12. Process according to any one of the preceding claims, characterized in that the fluoridated pigment thus obtained is then encapsulated with a coating layer based on at least one transparent oxide.

13. Coloured pigments based on rare-earth metal sesquisulphide(s), characterized in that they contain fluorine atoms.

14. Pigments according to claim 13, characterized in that the said fluorine atoms are distributed along a concentration gradient decreasing from the surface to the core of the said sesquisulphide.

15. Pigments according to either of claims 13 and 14, characterized in that the said fluorine atoms are mostly distributed at the outer periphery of the particles constituting the said sesquisulphide.

16. Pigments according to any one of claims 13 to 15, characterized in that the percentage by weight of the said fluorine atoms with respect to the said sesquisulphide does not exceed 10%.

17. Pigments according to claim 16, characterized in that the said percentage does not exceed 5%.

18. Pigments according to any one of claims 13 to 17, characterized in that the said fluorine atome are present in the said pigments in the form of fluoro or sulphofluoro compounds.

19. Pigments according to claim 18, characterized in that the said compounds are rare-earth metal fluorides and/or sulphofluorides.

20. Pigments according to any one of claims 13 to 19, characterized in that they additionally contain at least one alkali metal and/or alkaline-earth metal element (doping element), at least part of which is occluded in the crystal lattice of the said sesquisulphide.

21. Pigments according to claim 20, characterized in that the said doping element is essentially present in a form occluded in the crystal lattice of the said sesquisulphide.

22. Pigments according to either of claims 20 and 21, characterized in that the said doping element is present in the cationic gaps of the crystal lattice of the said sesquisulphide.

23. Pigments according to any one of claims 20 to 22, characterized in that the molar amount of doping element represents at least 0.1% of the molar amount of rare-earth metal(s).

24. Pigments according to claim 23, characterized in that the molar amount of doping element is between 5 and 50% of the molar amount of rare-earth metal(s).

25. Pigments according to any one of claims 20 to 24, characterized in that the said doping element is chosen, alone or as a mixture, from alkali metal elements.

26. Pigments according to claim 25, characterized in that the said doping element is sodium.

27. Pigments according to any one of claims 13 to 26, characterized in that the said rare-earth metal sesquisulphide is γ cubic cerium sesquisulphide Ce₂S₃.

28. Pigments according to claim 27, characterized in that they have an L* chromatic coordinate at least equal to 30.

29. Pigments according to claim 28, characterized in that the said L* chromatic coordinate is between 30 and 60.

30. Pigments according to any one of claims 27 to 29, characterized in that they have an a* chromatic coordinate equal to at least 30.

31. Pigments according to claim 30, characterized in that the said a* chromatic coordinate is between 35 and 65.

32. Pigments according to any one of claims 27 to 31, characterized in that they have a b* chromatic coordinate between 0 and 40.

33. Pigments according to any one of claims 13 to 32, characterized in that they have a mean particle size between 1 and 5 µm.

34. Pigments according to any one of claims 13 to 33, characterized in that they consist of γ cubic cerium sesquisulphide Ce₂S₃ doped with sodium.

35. Pigments according to any one of claims 13 to 34, characterized in that they additionally comprise a coating layer based on at least one transparent oxide deposited at their surface.

36. Pigments according to claim 35, characterized in that the said transparent oxide is chosen, alone or as a mixture, from the group consisting of silica, alumina, zirconia, zircon, titanium oxide and rare-earth metal oxides.

37. Pigments according to one of claims 35 or 36, characterized in that the said layer is based on silica.

38. Pigments according to claim 37, characterized in that the said layer consists solely of silica.

39. Pigments according to one of claims 35 to 38, characterized in that they consist of a substrate made of γ cubic cerium sesquisulphide Ce₂S₃ doped with sodium and containing fluorine atoms, which substrate is coated with a homogeneous layer of transparent silica.

40. Coloured pigments, characterized in that they are obtained by implementation of a process as defined in any one of claims 1 to 12.

41. Use of the coloured pigments defined in any one of claims 13 to 40 in plastics, paints, varnishes, rubbers, ceramics, glazes, papers, inks, cosmetics, dyes and laminated coatings.

42. Compositions of coloured material, especially of plastic, paint, varnish, rubber, ceramic, glaze, paper, ink, cosmetic product, dye and laminated coating type, characterized in that they comprise coloured pigments as defined in any one of claims 13 to 40.
